# EUROPÄISCHE PATENTANMELDUNG

(11) **EP 3 626 213 A1**
(43) Veröffentlichungstag der Anmeldung: **25.03.2020**
(21) Anmeldenummer: 19195044.3
(22) Anmeldetag: 03.09.2019
(51) Int. Cl.: A61F 9/007, A61F 9/008

(54) **CHIRURGISCHES INSTRUMENT, CHIRURGISCHE VORRICHTUNG, UND ELEKTRONISCHE STEUEREINRICHTUNG**

(30) Priorität: 21.09.2018 DE 202018105448 U
(71) Anmelder: Thyzel, Reinhardt, 90542 Eckental (DE)
(72) Erfinder: Thyzel, Reinhardt, 90542 Eckental (DE)
(74) Vertreter: Schröer, Gernot H.

(57) **Zusammenfassung**

Chirurgisches Instrument (1) für die Vitrektomie, insbesondere Vitrektomievorrichtung (1) bzw. Vitrektor (1), umfassend eine Hohlnadel (2), insbesondere Kanüle (2), mit einer zylindrischen Wandung (3), die einen Hohlraum (4, 8) der Hohlnadel (2) umschließt, wobei die Hohlnadel (2) an einem in Längsrichtung (L) der Hohlnadel (2) gelegenen distalen Ende (5) stirnseitig geschlossen ist, und die Hohlnadel (2) eine Vitrektomieöffnung (6) aufweist, deren Öffnungsnormale (7) radial oder quer zur Längsrichtung (L) orientiert ist, vom distalen Ende (5) einen vorgegebenen Mindestabstand aufweist, und deren Öffnungsquerschnittsfläche in Längsrichtung (L), oder quer zur Längsrichtung, oder in Umfangsrichtung (U) eine langgestreckte, zumindest abschnittsweise gekrümmte Form, insbesondere nach Art einer Ellipse ausgebildete Form, aufweist.

## Beschreibung

Die Erfindung betrifft insbesondere ein chirurgisches Instrument für die Vitrektomie, insbesondere eine Vitrektomievorrichtung bzw. einen Vitrektor, eine chirurgische Vorrichtung mit einem derartigen chirurgischen Instrument, sowie eine zugehörige elektronische Steuereinrichtung.

Bei der Vitrektomie werden Teile des Glaskörpers des Auges entfernt, was beispielsweise erforderlich sein kann bei Blutungstrübungen oder Netzhautablösung. Aufgrund der vergleichsweise festen Konsistenz der Glaskörpermasse ist es nicht in einfacher Weise möglich, diese in vivo abzusaugen.

Aus dem Stand der Technik, beispielsweise aus der WO 2015/135544 A1, sind Vitrektoren bekannt, die zur Abtrennung von Glaskörpermaterial in vivo mechanische bewegte Schneidelemente umfassen. Solche Vitrektoren können dahingehend nachteilig sein, dass mechanische Bewegungsenergie der bewegten Schneidelemente, z.B. in Form von Vibrationen, auf das Auge, insbesondere die Netzhaut, übertragen wird, und u.U. zu Schäden oder Beeinträchtigungen führt. Abgesehen davon wäre es wünschenswert, die Effizienz zum Entfernen von Glaskörpermaterial in vivo gegenüber den bekannten mechanischen Vitrektoren zu erhöhen.

Insbesondere ausgehend davon kann es als eine Aufgabe der Erfindung angesehen werden, ein chirurgisches Instrument für die Vitrektomie, insbesondere einen Vitrektor, eine chirurgische Vorrichtung, und eine entsprechende elektronische Steuereinrichtung bereitzustellen, die eine effiziente und schonende, insbesondere in vivo, Entfernung von Teilen des Glaskörpers des Auges ermöglichen.

Diese Aufgabe wird gelöst durch den Gegenstand der unabhängigen Schutzansprüche. Vorteilhafte Ausgestaltungen ergeben sich aus den abhängigen Schutzansprüchen. Ausgestaltungen ergeben sich ferner aus der nachfolgenden Beschreibung.

Nach Ausgestaltungen ist ein chirurgisches Instrument für die Vitrektomie, insbesondere eine Vitrektomievorrichtung bzw. ein Vitrektor, insbesondere eine Vitrektomienadel, vorgesehen.

Das chirurgische Instrument umfasst eine Hohlnadel, insbesondere Kanüle, mit einer zylindrischen Wandung, die einen Hohlraum, insbesondere inneren Hohlraum, der Hohlnadel umschließt. Die Hohlnadel ist an einem in Längsrichtung der Hohlnadel gelegenen distalen Ende stirnseitig, z.B. kuppel- oder kapselförmig, geschlossen.

Die Hohlnadel weist eine Vitrektomieöffnung auf, d.h. eine speziell zu Zwecken der Vitrektomie ausgebildete Öffnung.

Die Öffnungsnormale der Vitrektomieöffnung, insbesondere die Normale der in Draufsicht betrachteten Öffnungsquerschnittsfläche der Vitrektomieöffnung, ist radial oder quer zur Längsrichtung der Hohlnadel orientiert.

Ferner weist die Vitrektomieöffnung vom distalen Ende, insbesondere vom äußeren, stirnseitig geschlossenen distalen Ende der Hohlnadel, einen vorgegebenen Mindestabstand auf.

Weiterhin weist die Öffnungsquerschnittsfläche der Vitrektomieöffnung, insbesondere die in Draufsicht betrachtete Öffnungsquerschnittsfläche der Vitrektomieöffnung, in Längsrichtung, oder quer zur Längsrichtung, beispielsweise senkrecht zur Längsrichtung oder bezüglich der Längsrichtung in Umfangsrichtung der Hohlnadel betrachtet, eine langgestreckte, zumindest abschnittsweise gekrümmte Form, insbesondere eine nach Art einer (echten) Ellipse ausgebildete, langgestreckte Form, auf. Beispielsweise kann der durch die Vitrektomieöffnung definierte Umfangsrand, insbesondere bei Betrachtung in Draufsicht, eine langgestreckte, zumindest abschnittsweise gekrümmte Form, insbesondere eine nach Art einer (echten) Ellipse ausgebildete, langgestreckte Form, aufweisen.

Mit einer derartigen Vitrektomieöffnung ist ein Entfernen, insbesondere umfassend Zerkleinern bzw. Zerschneiden und Absaugen, von Teilen des Glaskörpers des Auges, insbesondere in vivo, in effizienter Weise möglich, beispielsweise in Verbindung mit einer durch Laserstrahlung bewirkten Zerkleinerung von Glaskörpermaterial, das über die Vitrektomieöffnung, z.B. bei Beaufschlagung des Inneren der Hohlnadel mit Unterdruck, ins Innere der Hohlnadel eingetreten ist.

In Ausgestaltungen kann Hohlnadel beispielsweise einwandig ausgebildet sein. Ferner kann die Vitrektomieöffnung in einer oder der, insbesondere einwandigen, Mantelwandung (oder: Zylinderwandung) der Hohlnadel ausgebildet sein.

Die Vitrektomieöffnung kann kommunizierend, insbesondere fluidtechnisch kommunizierend, mit einer sich an die Vitrektomieöffnung und an das distale Ende unmittelbar anschließende Kammer ausgebildet sein. Insbesondere mit einer derartigen Vitrektomieöffnung kann Glaskörpermaterial effizient und dennoch schonend in vivo entfernt, insbesondere abgesaugt, werden.

In Ausgestaltungen kann im Inneren der Hohlnadel ein zum Einleiten von Laserlicht, insbesondere von Laserpulsen, vorzugsweise von Nd:YAG Laserpulsen, in die Kammer ausgebildeter Lichtleiter, insbesondere eine Laserlicht-Leiteinrichtung oder eine Laseroptik, angeordnet sein. Der Lichtleiter kann insbesondere eine Lichtleitfaser und/oder eine Lichtleitoptik bzw. - faseroptik umfassen.

Die Lichtaustrittsseite oder -fläche des Lichtleiters oder die Lichtaustrittsseite oder -fläche einer Lichtoptik des Lichtleiters kann, gemäß Ausgestaltungen, der Kammer zugewandt sein, insbesondere derart, dass durch die Öffnung in die Kammer eingetretenes Glaskörpermaterial zum Zwecke der Zerkleinerung desselben mit Laserpulsen beaufschlagt werden kann. Beispielsweise kann die Lichtaustrittsseite derart angeordnet sein, dass Laserlicht, insbesondere Laserpulse, mit einer in Längsrichtung der Hohlnadel gerichteten Vorzugsrichtung abgegeben werden können, und dass entsprechend in Längsrichtung der Lichtaustrittsseite, z.B. in Richtung des distalen Endes, in der Kammer vorhandenes Glaskörpermaterial abgetrennt, zerkleinert oder zerschnitten werden kann.

In Ausgestaltungen kann die Hohlnadel im Inneren des Weiteren einen, sich vorzugsweise zumindest abschnittsweise parallel zum Lichtleiter erstreckenden, Absaugkanal (oder: Aspirationskanal) umfassen. Der Absaugkanal kann insbesondere derart ausgebildet sein, dass Glaskörpermaterial aus der Kammer, insbesondere aus dem inneren Hohlraum der Hohlnadel, nach Beaufschlagung mit Laserlicht, sprich nach erfolgter Abtrennung, abgesaugt werden kann. In Ausgestaltungen kann der Absaugkanal unmittelbar kommunizierend, d.h. fluidtechnisch kommunizierend, mit der Kammer ausgebildet sein.

In Ausgestaltungen kann der Abstand zwischen dem, dem stirnseitig geschlossenen distalen Ende zugewandten Öffnungsrand der Vitrektomieöffnung einerseits und dem äußeren stirnseitig geschlossenen distalen Ende der Hohlnadel andererseits im Bereich von 0,4 mm bis 1,8 mm, vorzugsweise im Bereich von 0,5 mm bis 1,6 mm, weiter vorzugsweise im Bereich von 0,55 bis 1,55, liegen.

In Ausgestaltungen kann der Abstand zwischen dem äußeren stirnseitig geschlossenen distalen Ende der Hohlnadel einerseits und dem in Längsrichtung der Hohlnadel gemessenem Mittelpunkt (oder: der Mittelachse) der Öffnungsquerschnittsfläche der Vitrektomieöffnung andererseits im Bereich von 0,4 mm bis 1,8 mm, vorzugsweise im Bereich von 0,5 mm bis 1,6 mm, weiter vorzugsweise im Bereich von 0,55 bis 1,55, liegen.

In Ausgestaltungen kann die parallel zur Längsrichtung gemessene Wandungsstärke des stirnseitig geschlossenen distalen Endes der Hohlnadel im Bereich von 0,25 mm bis 0,85 mm, vorzugsweise im Bereich von 0,3 mm bis 0,8 mm liegen.

Insbesondere die vorgenannten Abmessungen ermöglichen ein effizientes und gleichzeitig schonendes Absaugen von Glaskörpermaterial.

In Ausgestaltungen kann die Öffnungsquerschnittsfläche, d.h. die Querschnittsfläche der Vitrektomieöffnung, bzw. ein korrespondierenden Öffnungsrand, in Draufsicht (insbesondere: bei Draufsicht in radialer Richtung zur Längsachse hin) betrachtet an zumindest einem Längsende der Vitrektomieöffnung, vorzugsweise an beiden Längsenden der Vitrektomieöffnung eine abgerundete Form, vorzugsweise eine im Wesentlichen kreissegmentartige oder kreisbogenartige, insbesondere halbkreisbogenartige Form, aufweisen. Dabei soll der Begriff "Längsende" insbesondere ein in Längserstreckung der Vitrektomieöffnung gelegenes Ende, beispielsweise definiert durch den Rand der Vitrektomieöffnung in der Mantelfläche der Hohlnadel, bezeichnen.

In Ausgestaltungen können sich zwischen den Längsenden, d.h. zwischen den beiden in Längserstreckung der Vitrektomieöffnung gelegenen Enden, erstrechende Öffnungsränder zumindest abschnittsweise geradlinig und/oder parallel, vorzugsweise geradlinig parallel ausgebildet sein. Insbesondere können die zumindest abschnittsweise geradlinig ausgebildeten Öffnungsränder, bzw. entsprechende Öffnungsrandabschnitte, parallel zur Längsrichtung der Hohlnadel oder quer, insbesondere senkrecht, zur Hohlnadel verlaufen. Insbesondere die genannten Öffnungsgeometrien zeigen Vorteile hinsichtlich dem Eintreten von Glaskörpermaterial in die Kammer insbesondere in Kombination mit Laser-induzierter Zerkleinerung, und ermöglichen z.B. so ein effizientes Entfernen von Glaskörpermaterial in vivo.

In Ausgestaltungen kann die Öffnungsquerschnittsfläche, insbesondere in Draufsicht betrachtet, nach Art einer Ellipse, d.h. einer echten Ellipse, oder nach Art eines Langlochs ausgebildet sein.

In Ausgestaltungen kann die Hohlnadel (insbesondere zumindest teilweise, oder im Wesentlichen) aus Titan, insbesondere aus Titan Grade 4, bestehen, d.h. daraus (zumindest teilweise) gefertigt sein. Ferner kann die Hohlnadel, insbesondere die Wandung der Hohlnadel, als einstückige Einheit, beispielsweise aus einem einzigen Material ausgebildet, bzw. hergestellt sein.

In Ausgestaltungen kann die Hohlnadel eine Länge von zumindest oder maximal 25 mm, insbesondere von zumindest oder maximal 30 mm, aufweisen.

Ferner kann die Hohlnadel in Ausgestaltungen, insbesondere im Bereich der Vitrektomieöffnung, einen Außendurchmesser im Bereich von 0,35 mm bis 0,75 mm, vorzugsweise 0,4 mm bis 0,7 mm, insbesondere 0,65 mm, aufweisen.

Des Weiteren kann die Mantelwandung der Hohlnadel in Ausgestaltungen, insbesondere im Bereich der Vitrektomieöffnung, eine Dicke im Bereich von 0,1 mm bis 0,3 mm, vorzugsweise von 0,15 mm bis 0,25 mm, weiter vorzugsweise von 0,2 mm aufweisen.

Die genannten Hohlnadelabmessungen eignen sich insbesondere für eine effiziente und schonende Durchführung einer Vitrektomie.

In Ausgestaltungen kann die Vitrektomieöffnung, insbesondere deren Öffnungsquerschnittsfläche, eine Länge im Bereich von 0,5 mm bis 0,8 mm, insbesondere 0,55 mm bis 0,75 mm, insbesondere 0,65 oder 0,6 mm, und eine Breite im Bereich von 0,25 mm bis 0,35 mm, insbesondere 0,3 mm, aufweisen. Entsprechende Vitrektomieöffnungen ermöglichen, insbesondere in Verbindung mit Laserbeaufschlagung des Glaskörpermaterials in der Kammer, beispielsweise mit einer Vorzugslaserausbreitungsrichtung parallel zur Längsachse, eine besonders effiziente Entfernung von Glaskörpermaterial, insbesondere hinsichtlich Abtragraten bzw. Absaugraten.

In Ausgestaltungen kann die Außenfläche des stirnseitig geschlossenen distalen Endes der Hohlnadel zumindest abschnittsweise, beispielsweise kuppel- oder kapselartig, gekrümmt sein. Ferner kann die Außenfläche in Ausgestaltungen zumindest abschnittsweise einen Krümmungsradius im Bereich von 0,15 mm bis 0,35 mm, vorzugsweise 0,2 mm bis 0,3 mm aufweisen. Solche Nadelgeometrien eignen sich insbesondere zur schonenden Durchführung einer Vitrektomie.

In Ausgestaltungen kann der Öffnungsrand der Hohlnadel, insbesondere in Draufsicht betrachtet, zumindest abschnittsweise gekrümmt sein mit einem Krümmungsradius im Bereich von 0,1 mm bis 0,2 mm, vorzugsweise 0,15 mm. Solche Randgeometrien haben sich insbesondere geeignet dahingehend erwiesen, dass bei einer in vivo Vitrektomie, Glaskörpermaterial vergleichsweise effizient in die sich an die Vitrektomieöffnung anschließende Kammer gesaugt und entfernt werden kann.

In Ausgestaltungen kann eine chirurgische Vorrichtung, insbesondere eine Vitrektomie-Vorrichtung, vorgesehen sein, die ein chirurgisches Instrument nach einer der hierin beschrieben Ausgestaltungen, insbesondere gemäß einer Ausgestaltung der anliegenden Schutzansprüche, umfasst.

Die chirurgische Vorrichtung kann eine Laserlichtquelle, insbesondere eine Nd:YAG Laserlichtquelle, beispielsweise eine Q-switched Nd:YAG Laserquelle, zur Einkopplung von Laserlicht, insbesondere Laserpulsen, in den Hohlraum, insbesondere in die Kammer, der Hohlnadel, umfassen.

Zur Einkopplung des Laserlichts in den Hohlraum, z.B. in die fluidtechnisch mit der Vitrektomieöffnung (unmittelbar) kommunizierend ausgebildete Kammer, kann die Vorrichtung einen Lichtleiter, insbesondere ein Laserlichtleitelement umfassen. Der Lichtleiter kann beispielsweise zumindest abschnittsweise im Inneren der Hohlnadel angeordnet sein.

Die Laserlichtquelle, insbesondere eine Steuereinheit zur Steuerung der Laserlichtquelle, kann in Ausgestaltungen dazu eingerichtet sein, Laserlicht mit einer Pulsfrequenz im Bereich von 40 Hz bis 60 Hz zu erzeugen. Die Laserlichtquelle kann lichttechnisch mit dem Lichtleiter oder einer geeigneten Lichtoptik gekoppelt sein oder koppelbar sein, derart, dass von der Laserlichtquelle erzeugtes Laserlicht, insbesondere Laserpulse, über den Lichtleiter oder die Lichtoptik in die Kammer geleitet werden kann/können.

Die Vorrichtung kann in Ausgestaltungen des Weiteren eine mit dem inneren Hohlraum der Hohlnadel gekoppelte oder koppelbare Absaugeinheit (insbesondere: Aspirationseinheit) umfassen. Die Absaugeinheit kann in Ausgestaltungen derart eingerichtet und betreibbar sein, dass der Hohlraum der Hohlnadel mit Unterdruck beaufschlagbar ist. Insbesondere kann die Absaugeinheit derart eingerichtet sein, dass der Hohlraum, insbesondere die mit der Vitrektomieöffnung fluidtechnisch gekoppelte Kammer, mit Unterdruck im Bereich von 0,2 bar (20 kPa) bis 0,4 bar (40 kPa), vorzugsweise von 0,25 bar (25 kPa) bis 0,35 bar (35 kPa), weiter vorzugsweise von 0,26 bar (26 kPa) bis 0,33 bar (33 kPa) beaufschlagbar ist.

In Ausgestaltungen kann eine elektronische Steuereinrichtung mit einer Steuerschnittstelle zur steuerungstechnischen Kopplung mit einem chirurgischen Instrument nach einem der hierin beschriebenen Ausgestaltungen, insbesondere nach einer der Ausgestaltungen der Schutzansprüche, und/oder mit einer chirurgischen Vorrichtung nach einer hierin beschriebenen Ausgestaltung, insbesondere einer der Ausgestaltungen der Schutzansprüche, vorgesehen sein.

Die Steuereinrichtung kann in Ausgestaltungen Steuermittel umfassen, insbesondere in Form von Hardware und/oder in Form von auf oder in einem Speicher gespeicherten Befehlen zur Ausführung durch eine elektronische Rechner- oder Datenverarbeitungseinheit, deren Ausführung bei Betrieb der Steuereinrichtung bei dem chirurgischen Instrument bzw. der chirurgischen Vorrichtung zumindest einen der folgenden Verfahrensschritte bewirken:
- Erzeugen von Laserpulsen, insbesondere mit einer Laserpulsfrequenz im Bereich von 40 Hz bis 60 Hz, wobei die Laserpulse über einen Lichtleiter oder eine Lichtoptik in den inneren Hohlraum der Hohlnadel, insbesondere die Kammer, eingekoppelt werden, und an einer Lichtaustrittsseite des Lichtleiters bzw. der Lichtoptik austreten, beispielsweise mit einer Vorzugsrichtung parallel zur Längsrichtung der Hohlnadel, derart, dass in einer bzw. der im Inneren der Hohlnadel ausgebildeten Kammer befindliches Glaskörpermaterial des Auges durch Einwirkung der Laserpulse zerkleinert, insbesondere zerschnitten, wird. Dabei ist die Kammer mit einer gemäß einer hierin beschriebenen Ausgestaltung, insbesondere einer gemäß einer anspruchsgemäßen Ausgestaltung, ausgebildeten Vitrektomieöffnung fluidtechnisch kommunizierend ausgebildet.
- Beaufschlagen des inneren Hohlraums der Hohlnadel, insbesondere der Kammer, mit Unterdruck, insbesondere im Bereich von 0,2 bar bis 0,4 bar, vorzugsweise von 0,25 bar bis 0,35 bar, weiter vorzugsweise von 0,26 bar bis 0,33 bar, derart, dass das durch die Laserpulse zerkleinerte Glaskörpermaterial, insbesondere durch die Wirkung des Unterdrucks abgesaugt, und gleichzeitig weiteres Glaskörpermaterial in die Kammer eintritt wenn die entsprechend nach einer hierin beschriebenen Ausgestaltung, insbesondere einer anspruchsgemäßen Ausgestaltung, ausgebildete Vitrektomieöffnung, insbesondere in vivo, im Glaskörper eines Auges, z.B. eines menschlichen oder tierischen Auges, positioniert ist.

Nachfolgend werden mit Bezug zu den anhängenden Zeichnungen Ausführungsbeispiele der Erfindung näher beschrieben. Es zeigen:
- FIG. 1: einen Vitrektor in einer ersten Ausführungsform in Draufsicht;
- FIG. 2: den Vitrektor der FIG. 1 im Querschnitt;
- FIG. 3: einen Vitrektor in einer zweiten Ausführungsform in Draufsicht;
- FIG. 4: den Vitrektor der FIG. 3 im Querschnitt
- FIG. 5: einen Vitrektor in einer dritten Ausführungsform in Draufsicht;
- FIG. 6: ein Detail des Vitrektors der FIG. 5;
- FIG. 7: einen Querschnitt des Vitrektors nach FIG. 5; und
- FIG. 8: eine perspektivische Teil-Darstellung des Vitrektors der FIG. 6.

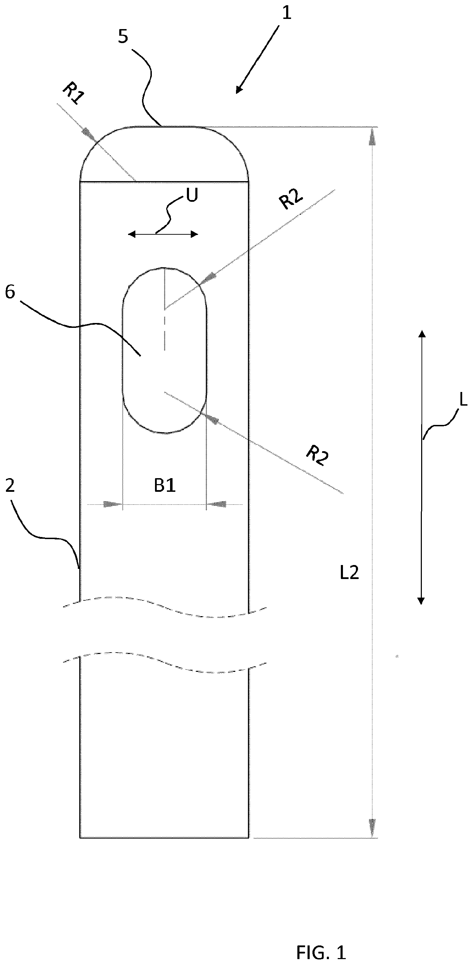

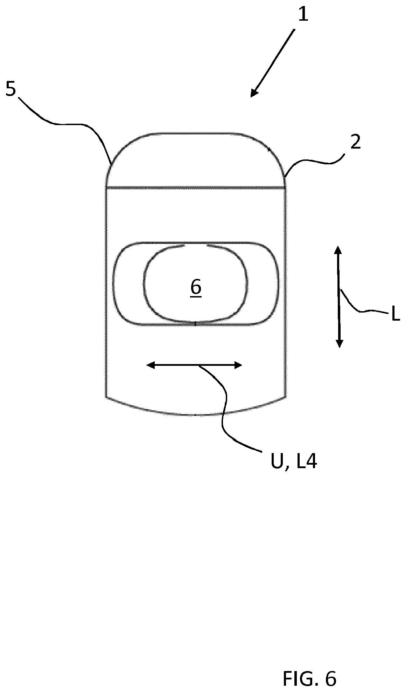

Einander entsprechende Teile und Größen sind in den FIG 1 bis 7 mit denselben Bezugszeichen versehen.

Die FIG. 1 bis 8 zeigen Ausführungsbeispiele für ein chirurgisches Instrument, insbesondere einen Vitrektor 1 bzw. eine Vitrektomienadel zur Durchführung einer Vitrektomie, insbesondere am Auge in vivo.

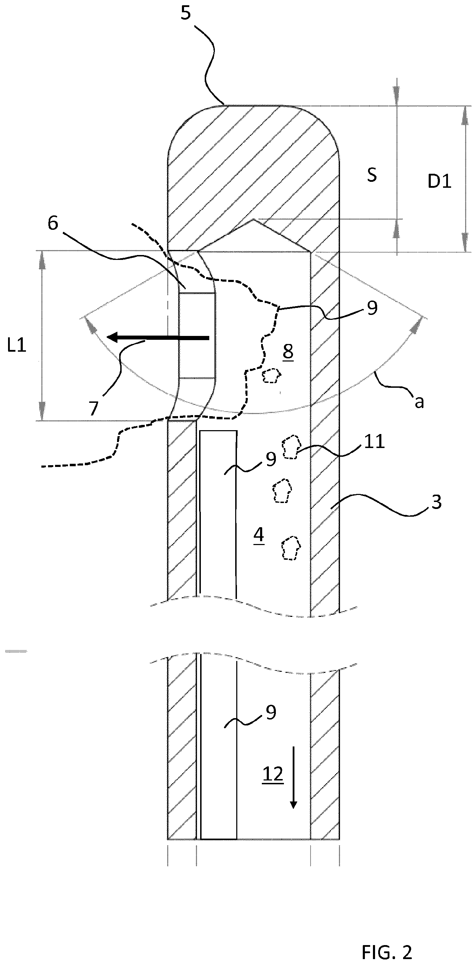

Der Vitrektor 1 nach FIG. 1 umfasst eine Hohlnadel 2, beispielsweise nach Art. einer Kanüle. Die Hohlnadel 2 umfasst, was insbesondere in Zusammensicht mit FIG. 2 deutlich wird, eine zylindrische Wandung 3, die einen Hohlraum 4 der Hohlnadel 2 umschließt.

Die Hohlnadel 2 ist an einem in Längsrichtung L der Hohlnadel 2 gelegenen distalen Ende 5 stirnseitig geschlossen.

Die Hohlnadel 2 weist ferner eine Vitrektomieöffnung 6 auf, wobei die Öffnungsnormale 7 der Vitrektomieöffnung 6 radial zur Längsrichtung L orientiert ist. Vom distalen Ende 5 ist die Vitrektomieöffnung 6 im gegebenen Ausführungsbeispiel 0,42 mm entfernt. Genauer beträgt im vorliegenden Ausführungsbeispiel der Abstand D1 zwischen dem distalen Ende 5 einerseits und dem, dem distalen Ende 5 zugewandt gelegenen Öffnungsrand der Vitrektomieöffnung andererseits 0,42 mm.

Die Öffnungsquerschnittsfläche der Vitrektomieöffnung 6, insbesondere in Draufsicht nach FIG. 1 betrachtet, weist im vorliegenden Ausführungsbeispiel in Längsrichtung L, eine langgestreckte, abschnittsweise gekrümmte Form, insbesondere nach Art eines Langlochs auf. Insbesondere sind beiderseitige Seiten des Öffnungsrands halbkreisförmig ausgebildet, und durch geradlinig verlaufende Randabschnitte verbunden.

Abmessungen und geometrische Einzelheiten für den Vitrektor 1, bzw. die Hohlnadel 2 sowie für die Vitrektomieöffnung 6 ergeben sich insbesondere aus FIG. 1 und 2.

So hat bei der gegebenen Ausgestaltung die Vitrektomieöffnung 6 in Umfangsrichtung U gemessen eine Breite B1 von 0,3 mm, und in Längsrichtung L gemessen eine Länge L1 von 0,6 mm. Die Gesamtlänge L2 der Hohlnadel beträgt ca. 25 mm.

Krümmungsradien R2 können für die halbkreisförmig ausgebildeten Öffnungsränder beispielsweise 0,15 mm betragen. Abrundungen im Bereich des distalen Endes 5 können beispielsweise Krümmungsradien R2 von 0,2 mm aufweisen. Beispielsweise kann die äußere Form des distalen Endes 5 kuppelartig ausgebildet sein. Andere Formen kommen jedoch ebenfalls in Frage.

Wie aus der Querschnittsdarstellung der FIG. 2 ersichtlich ist, kann die Dicke S der Wandung 3 der Hohlnadel 2 am distalen Ende 5, gemessen in Richtung der Längsachse der Hohlnadel 2 im Bereich von 0,3 mm liegen.

Die Hohlnadel 2 als solche kann beispielsweise, insbesondere einstückig, aus Vollmaterial Ti-Grade 4 hergestellt sein, wobei der Hohlraum 4 durch Bohren hergestellt sein kann, so dass bei einem Außendurchmesser von 0,6 mm sich ein Innendurchmesser von 0,4 mm ergibt.

Durch Verwendung eines Bohrers mit konischer Bohrspitze ergibt sich am distal endseitig gelegenen Ende des Hohlraums 4 ein Öffnungswinkel a, der im vorliegenden Beispiel 120 Grad beträgt. Jedoch kommen auch andere Öffnungswinkel in Frage. Insbesondere ist es, beispielsweise bei Verwendung eines Bohrers mit nichtkonischer Bohrspitze oder bei anderen Verfahren zur Erzeugung des Hohlraums, auch möglich, dass die distal endseitige Innenwand des Hohlraums gerade, insbesondere senkrecht zur Längsrichtung L, verläuft, oder konvex oder konkav gekrümmt ist.

Wie sich aus FIG. 2 ergibt ist die Hohlnadel 2 einwandig und die Vitrektomieöffnung 6 ist in der Mantelwandung, d.h. der zylindrischen Wandung 3, der Hohlnadel 2 ausgebildet.

Die Vitrektomieöffnung 6 ist fluidtechnisch kommunizierend unmittelbar anschließend zu einer im Inneren der Hohlnadel 2 gelegenen Kammer 8 ausgebildet. Oder anders ausgedrückt, im Bereich der Vitrektomieöffnung 6 bildet der innere Hohlraum 4 der Hohlnadel 2 eine Kammer 8, die dazu eingerichtet ist, durch die Vitrektomieöffnung eintretendes (in FIG. 2 durch gepunktete Linie angedeutetes) Glaskörpermaterial 9, z.B. bei in vivo Vitrektomie des Auges, aufzunehmen.

Durch eine Lichtoptik, insbesondere eine lichtoptische Faser 10, kann mittels einer (nicht gezeigten) Laservorrichtung Laserlicht, insbesondere in Form von Laserpulsen, beispielsweise mit einer Frequenz von 40 Hz bis 60 Hz, in die Kammer 8 eingestrahlt werden, beispielsweise mit einer parallel zur Längsrichtung verlaufenden Vorzugsrichtung. Durch die Einwirkung des Laserlichts kann das in der Kammer 8 befindliche Glaskörpermaterial 9 zersetzt, insbesondere "zerschnitten", werden. Insbesondere können Glaskörpermaterialstücke 11 abgetrennt werden.

Abgetrennte Glaskörpermaterialstücke 11 können durch einen im Hohlraum 4 der Hohlnadel 2 ausgebildeten Absaugkanal 12 z.B. in Richtung des dem Bezugszeichen 12 zugeordneten Pfeils, d.h. vom distalen Ende weg, abtransportiert werden.

Zum Absaugen der Glaskörpermaterialstücke 11 kann an der Hohlnadel 2, d.h. im Hohlraum 4, ein Unterdruck von z.B. 200 bis 250 mmHg (ca. 26,7 kPa bis 33,3 kPa) angelegt werden. Der Unterdruck bewirkt ferner beispielsweise, dass Glaskörpermaterial 9, z.B. bei der in vivo Vitrektomie, durch die Vitrektomieöffnung 6 in die Kammer 8 gesaugt wird, so dass dieses mit Laserstrahlung, insbesondere Laserpulsen, beaufschlagt werden kann. Die Laserstrahlung bzw. die Laserpulse können wie erwähnt beispielsweise mit einem Nd:YAG Laser erzeugt werden.

Beim Ausführungsbeispiel der FIG. 2 ist die Lichtaustrittsseite bzw. Lichtaustrittsfläche des Lichtleiters 9 bzw. der Lichtoptik der Kammer 8 in Richtung des distalen Endes 5 zugewandt.

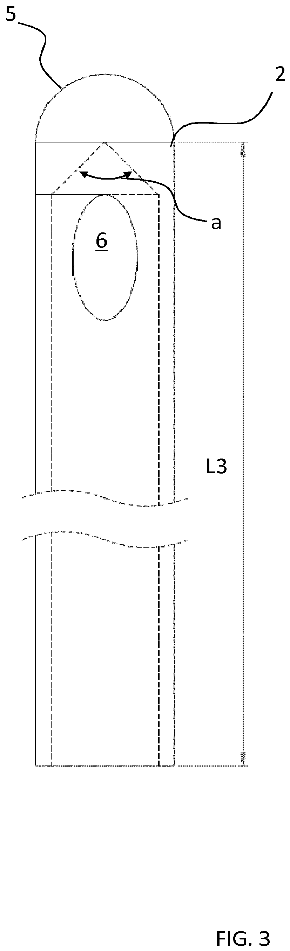

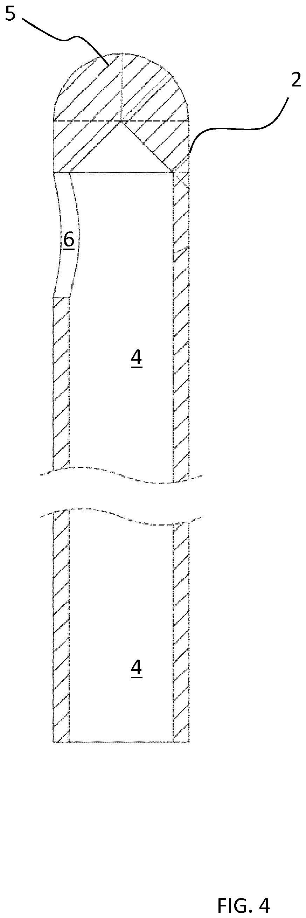

FIG. 3 und 4 zeigen ein zweites Ausführungsbeispiel einer Hohlnadel 2. Im Nachfolgenden soll lediglich auf die wesentlichen Unterschiede zur Hohlnadel der FIG. 1 und 2 eingegangen werden. Sofern sich aus der nachfolgenden Beschreibung nichts anderes ergibt, können im Zusammenhang mit dem zweiten und dritten Ausführungsbeispiel nicht näher diskutierte Elemente und Komponenten, oder in den Figuren nicht dargestellte Elemente und Komponenten, grundsätzlich gleich wie bei der ersten Ausgestaltung ausgebildet und angeordnet sein können.

Insbesondere unterscheidet sich die Vitrektomieöffnung 6 der FIG. 3 von der der FIG. 1 in der konkreten geometrischen Form, insbesondere der Form der Öffnungsquerschnittsfläche. Während die Vitrektomieöffnung 6 der FIG. 1 nach Art eines Langlochs ausgebildet ist, ist die Vitrektomieöffnung 6 der FIG. 3 und 4, in Draufsicht betrachtet, in Richtung der Längsachse L der Hohlnadel 2 als Ellipse bzw. ellipsenförmig ausgebildet. Eine solche Form hat sich neben der in FIG. 1 und 2 gezeigten Form ebenfalls als besonders geeignet für eine effiziente Durchführung einer Vitrektomie herausgestellt.

Unterschiede bestehen noch im Öffnungswinkel a der den Hohlraum 4 bildenden Längsbohrung im Bereich des distalen Endes 5. Der Öffnungswinkel a beträgt im Beispiel der FIG. 3 beispielhaft etwa 90 Grad, kann jedoch auch verschieden sein. Beispielsweise kann die stirnseitige Endfläche des Hohlraums 4 bzw. der Kammer 8 am distalen Ende 5 beispielsweise auch gerade (z.B. quer oder senkrecht zur Längsachse, verlaufen, oder konvex oder konkav gekrümmt sein.

Ein weiterer Unterschied besteht in der äußeren Form des distalen Endes 5 der Hohlnadel 2. Im Ausführungsbeispiel der FIG. 3 und 4 ist die Form des äußeren distalen Endes 5 kuppel- oder kapselfömig, im Querschnitt insbesondere halbkreisförmig, oder kreissegmentartig ausgebildet. Die Länge L3 des zylinderförmigen Abschnitts der Hohlnadel 2, d.h. ohne gekrümmtes distales Ende, kann z.B. etwa 25 mm betragen.

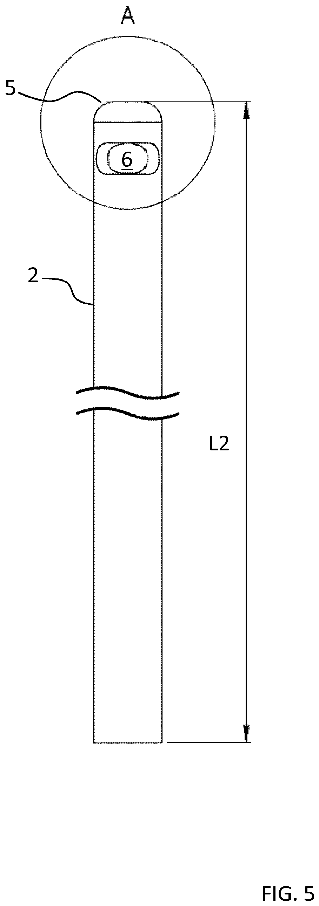

FIG. 5 bis FIG. 8 zeigen Ansichten eines drittes Ausführungsbeispiels eines Vitrektors 1, bzw. einer Hohlnadel 2. Der Unterschied des Vitrektors 1 zum ersten und zweiten Ausführungsbeispiel liegt insbesondere in der Orientierung bzw. geometrischen Ausdehnung der Vitrektomieöffnung 6.

Im dritten Ausführungsbeispiel weist die Vitrektomieöffnung 6, wie bei den anderen Ausführungsbeispielen, eine vorteilhafte (insbesondere in Draufsicht betrachtet) längsgestreckte Form auf, jedoch ist, im Unterschied zum ersten und zweiten Ausführungsbeispiel, die in Umfangsrichtung U gemessene Ausdehnung der Vitrektomieöffnung größer als deren Ausdehnung in Längsrichtung L.

Die in Draufsicht betrachtete Öffnungsfläche der Vitrektomieöffnung 6 entspricht in etwa der Form eines in Umfangrichtung U orientierten Langlochs mit zumindest an den gegenüberliegenden Enden gekrümmten, beispielsweise kreisartig oder alternativ auch kreisförmig gekrümmten, Öffnungsrändern.

Auch die in FIG. 5 bis 8 dargestellte Form der Vitrektomieöffnung 6 ermöglicht eine besonders effiziente und schonende Vitrektomie.

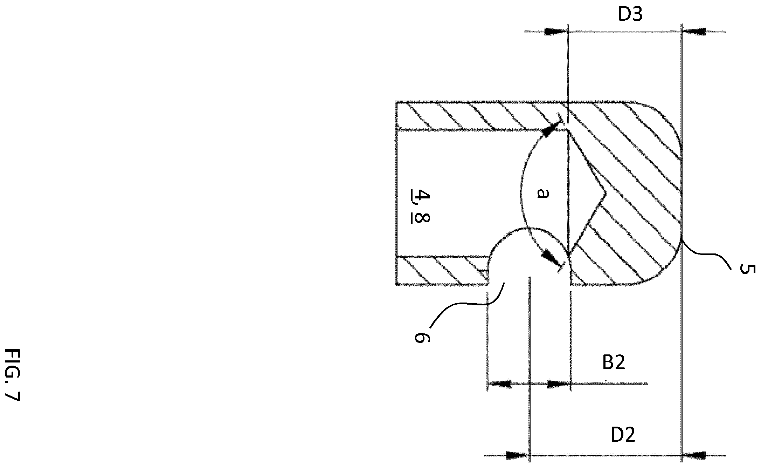

Einige beispielhafte Abmessungen sind aus FIG. 7 ersichtlich. So kann der Innendurchmesser des zylindrischen Abschnitts der Hohlnadel 2 beispielsweise 0,45 mm betragen. Die in Längsrichtung L gemessene Breite B2 der Vitrektomieöffnung 6 kann z.B. 0,3 mm betragen, wobei die in Umfangsrichtung U gemessene Länge L4 beispielsweise etwa 0,6 mm betragen kann.

Ein Abstand D2 Dzwischen äußerem distalen Ende 5 der Hohlnadel 2 und der Mittelachse der Vitrektomieöffnung 6, insbesondere der Mittelachse der Öffnungsfläche der Vitrektomieöffnung 6, kann beispielsweise 0,55 mm betragen, wobei ein Abstand D3 zwischen äußerem distalen Ende 5 der Hohlnadel 2 und dem, dem distalen Ende 5 zugewandten Öffnungsrand beispielsweise 0,41 mm betragen kann.

Ein Öffnungswinkel a der Längsbohrung am distalen Ende 5 kann beispielsweise 118 Grad betragen. Jedoch kann der Öffnungswinkel a, z.B. je nach verwendetem Bohrer, auch anders sein. Die am distalen Ende 5 gelegene Endfläche des Hohlraums 4 bzw. der Kammer 8 kann alternativ auch gerade bzw. eben, oder gekrümmt, z.B. konkav oder konvex gekrümmt sein.

Obgleich im zweiten und dritten Ausführungsbeispiel nicht alle im Zusammenhang mit dem ersten Ausführungsbeispiel gezeigt sind, z.B. die lichtoptische Faser 10, sind diese bei funktionsfähiger Ausbildung der Hohlnadeln 2 entsprechend vorhanden.

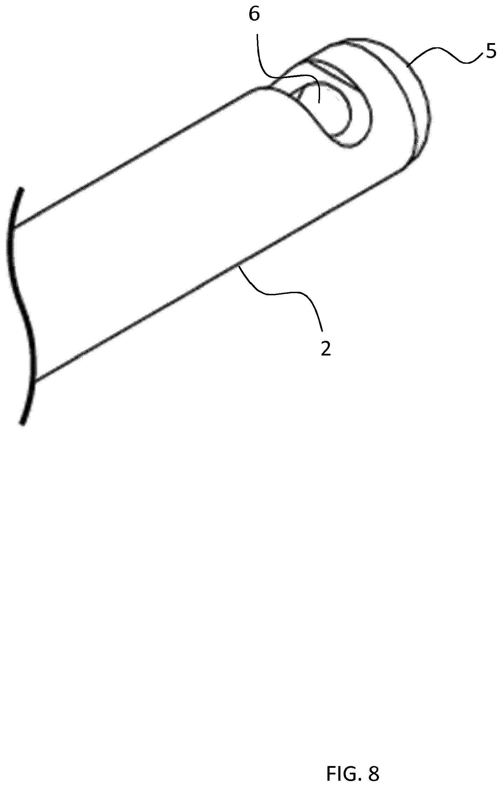

FIG. 8 zeigt schließlich noch eine perspektivische Darstellung der Hohlnadel 2 des dritten Ausführungsbeispiels, aus der die Vitrektomieöffnung 6 mit ihrer sich in Umfangsrichtung U erstreckenden erstreckenden Längsachse deutlich erkennbar ist.

Insbesondere kann mit einer Vitrektomieöffnung mit einer in Umfangsrichtung U sich erstreckenden Längsachse, d.h. die Länge der Vitrektomieöffnung 6 in Umfangsrichtung ist größer ist als die Breite in Richtung der Längsachse L, in Kombination mit Laserpulsen, die im Wesentlichen in Längsrichtung in die Kammer 8 abgegeben werden, eine vergleichsweise effizientes Vitrektomieergebnis, insbesondere hinsichtlich der Abtragraten, erzielt werden.

Entsprechend den drei Ausführungsbeispielen kann ein hierin vorgeschlagener Vitrektor 1 insbesondere eine Öffnungsquerschnittsfläche aufweisen, die in Draufsicht betrachtet an zumindest einem Längsende, vorzugsweise an beiden Längsenden eine abgerundete Form, vorzugsweise eine im Wesentlichen kreissegmentartige oder kreisbogenartige, insbesondere halbkreisbogenartige Form, aufweist, und/oder es kann vorgesehen sein, dass sich zwischen den Längsenden erstrechende Öffnungsränder zumindest abschnittsweise geradlinig und/oder parallel, vorzugsweise geradlinig parallel ausgebildet sind. Insbesondere kann Öffnungsquerschnittsfläche in Draufsicht betrachtet nach Art einer Ellipse, oder nach Art eines Langlochs, insbesondere nach Art eines von einem (durch gerade Öffnungsränder definierten) Schlitz verschiedenen Langlochs ausgebildet sein. Die Vitrektomieöffnung kann beispielsweise durch Bohren oder Fräser hergestellt sein.

Ferner ist es möglich, dass der Öffnungsrand der Hohlnadel 2, insbesondere in Draufsicht betrachtet, zumindest abschnittsweise gekrümmt ist mit einem Krümmungsradius R2 im Bereich von 0,1 mm bis 0,2 mm, vorzugsweise 0,15 mm.

Die zu den ersten bis dritten Ausführungsbeispielen gezeigten Hohlnadeln 2 können Teil einer chirurgischen Vorrichtung sein, die des Weiteren, wie weiter oben beschrieben, eine Laserlichtquelle, insbesondere eine Nd:YAG La-serlichtquelle, zur Einkopplung von Laserlicht, insbesondere Laserpulsen, in den Hohlraum 4 bzw. die Kammer 8 der Hohlnadel 2, insbesondere über die zumindest abschnittsweise im Inneren der Hohlnadel 2 angeordnete lichtoptische Faser 10, aufweisen. Die Laserlichtquelle, kann beispielsweise eine Steuereinheit zur Steuerung der Laserlichtquelle aufweisen, die dazu eingerichtet ist, Laserlicht mit einer Pulsfrequenz im Bereich von 40 Hz bis 60 Hz zu erzeugen und in die Kammer 8 einzuspeisen.

Ferner kann eine solche Vorrichtung eine (nicht gezeigte), mit dem inneren Hohlraum 4 und der Kammer 8 der Hohlnadel 2 gekoppelte oder koppelbare Absaugeinheit umfassen. Die die Absaugeinheit kann z.B. derart eingerichtet und betreibbar sein, dass der Hohlraum 4 insbesondere die Kammer 8 der Hohlnadel 2 mit Unterdruck beaufschlagbar ist, insbesondere mit einem Unterdruck im Bereich von 200 mmHg bis 250 mmHg.

Ferner kann eine elektronische Steuereinrichtung mit einer Steuerschnittstelle zur steuerungstechnischen Kopplung mit einem entsprechenden chirurgischen Instrument vorgesehen sein, wobei die Steuereinrichtung Steuermittel umfassen kann, deren Ausführung bei Betrieb der Steuereinrichtung bei dem chirurgischen Instrument 1 bzw. der chirurgischen Vorrichtung zumindest einen der bereits weiter oben beschriebenen Verfahrensschritte bewirken kann.

Insgesamt zeigt sich, dass das hierin vorgeschlagene chirurgische Instrument, die chirurgische Vorrichtung bzw. die Steuereinrichtung das zu Grunde liegende Problem lösen.

### Bezugszeichenliste

- 1: Vitrektor
- 2: Hohlnadel
- 3: zylindrische Wandung
- 4: Hohlraum
- 5: distales Ende
- 6: Vitrektomieöffnung
- 7: Öffnungsnormale
- 8: Kammer
- 9: Glaskörpermaterial
- 10: lichtoptische Faser
- 11: Glaskörpermaterialstücke
- 12: Absaugkanal

- a: Öffnungswinkel
- Bx: Breiten
- Dx: Abstände
- L: Längsrichtung
- Lx: Längen
- U: Umfangsrichtung
- Rx: Krümmungsradien
- S: Dicke

## Patentansprüche

1. Chirurgisches Instrument (1) für die Vitrektomie, insbesondere Vitrektomievorrichtung (1) bzw. Vitrektor (1), umfassend
eine Hohlnadel (2), insbesondere Kanüle (2), mit einer zylindrischen Wandung (3), die einen Hohlraum (4, 8) der Hohlnadel (2) umschließt, wobei
die Hohlnadel (2) an einem in Längsrichtung (L) der Hohlnadel (2) gelegenen distalen Ende (5) stirnseitig geschlossen ist,
und die Hohlnadel (2) eine Vitrektomieöffnung (6) aufweist, deren Öffnungsnormale (7) radial oder quer zur Längsrichtung (L) orientiert ist, vom distalen Ende (5) einen vorgegebenen Mindestabstand aufweist, und deren Öffnungsquerschnittsfläche in Längsrichtung (L), oder quer zur Längsrichtung, oder in Umfangsrichtung (U) eine langgestreckte, zumindest abschnittsweise gekrümmte Form, insbesondere nach Art einer Ellipse ausgebildete Form, aufweist.

2. Chirurgisches Instrument (1) nach Anspruch 1, wobei die Hohlnadel (2) einwandig ausgebildet ist, und/oder die Vitrektomieöffnung (6) in der Mantelwandung (3) der Hohlnadel (2) ausgebildet ist, und kommunizierend mit einer sich an die Vitrektomieöffnung (6) und an das distale Ende (5) unmittelbar anschließende Kammer (8) ausgebildet ist.

3. Chirurgisches Instrument (1) nach Schutzanspruch 2, wobei im Inneren der Hohlnadel (2) ein zum Einleiten von Laserlicht, insbesondere von Laserpulsen, vorzugsweise von Nd:YAG Laserpulsen, in die Kammer (8) ausgebildeter Lichtleiter (10) angeordnet ist, wobei die Lichtaustrittsseite des Lichtleiters (10) oder einer Lichtoptik des Lichtleiters (10) der Kammer (8) zugewandt ist, wobei die Hohlnadel (2) im Inneren des Weiteren einen, sich vorzugsweise zumindest abschnittsweise parallel zum Lichtleiter (10) erstreckenden, Absaugkanal (12) umfasst, der insbesondere ausgebildet ist zum Absaugen von Glaskörpermaterial (11) aus der Kammer (8) nach Beaufschlagung mit Laserpulsen, wobei der Absaugkanal (12) unmittelbar kommunizierend mit der Kammer (8) ausgebildet ist.

4. Chirurgisches Instrument (1) nach einem der Schutzansprüche 1 bis 3, wobei der Abstand (D1, D3) zwischen dem, dem stirnseitig geschlossenen distalen Ende (5) zugewandten Öffnungsrand der Vitrektomieöffnung (6) und dem äußeren stirnseitig geschlossenen distalen Ende (5) der Hohlnadel (2) im Bereich von 0,4 mm bis 1,8 mm, vorzugsweise im Bereich von 0,5 mm bis 1,6 mm, weiter vorzugsweise im Bereich von 0,55 bis 1,55, liegt, und/oder wobei der Abstand (D2) zwischen dem äußeren stirnseitig geschlossenen distalen Ende (5) der Hohlnadel (2) und dem in Längsrichtung (L) der Hohlnadel (2) gemessenem Mittelpunkt der Öffnungsquerschnittsfläche der Vitrektomieöffnung (6) im Bereich von 0,4 mm bis 1,8 mm, vorzugsweise im Bereich von 0,5 mm bis 1,6 mm, weiter vorzugsweise im Bereich von 0,55 bis 1,55, liegt, und/oder wobei die parallel zur Längsrichtung (L) gemessene Wandungsstärke (S) des stirnseitig geschlossenen distalen Endes (5) der Hohlnadel (2) im Bereich von 0,25 mm bis 0,85 mm, vorzugsweise im Bereich von 0,3 mm bis 0,8 mm liegt.

5. Chirurgisches Instrument (1) nach einem der Schutzansprüche 1 bis 4 wobei die Öffnungsquerschnittsfläche in Draufsicht betrachtet an zumindest einem Längsende, vorzugsweise an beiden Längsenden eine abgerundete Form, vorzugsweise eine im Wesentlichen kreissegmentartige oder kreisbogenartige, insbesondere halbkreisbogenartige Form, aufweist, und/oder wobei sich zwischen den Längsenden erstrechende Öffnungsränder zumindest abschnittsweise geradlinig und/oder parallel, vorzugsweise geradlinig parallel ausgebildet sind.

6. Chirurgisches Instrument (1) nach Schutzanspruch 5, wobei die Öffnungsquerschnittsfläche, insbesondere in Draufsicht betrachtet, nach Art einer Ellipse, oder nach Art eines Langlochs ausgebildet ist.

7. Chirurgisches Instrument (1) nach einem der Schutzansprüche 1 bis 6 wobei die Hohlnadel (2) aus Titan, insbesondere aus Titan Grade 4, besteht oder gefertigt ist, und/oder wobei die Hohlnadel (2) eine Länge (L2) von zumindest 25 mm, insbesondere 30 mm, aufweist, und/oder wobei die Hohlnadel (2), insbesondere im Bereich der Vitrektomieöffnung (6), einen Außendurchmesser im Bereich von 0,35 mm bis 0,75 mm, vorzugsweise 0,4 mm bis 0,7 mm, insbesondere 0,65 mm, aufweist, und/oder wobei die Mantelwandung (3) der Hohlnadel (2), insbesondere im Bereich der Vitrektomieöffnung (6), eine Dicke im Bereich von 0,1 mm bis 0,3 mm, vorzugsweise von 0,15 mm bis 0,25 mm, weiter vorzugsweise von 0,2 mm aufweist.

8. Chirurgisches Instrument (1) nach einem der Schutzansprüche 1 bis 7 wobei die Vitrektomieöffnung (6), insbesondere deren Öffnungsquerschnittsfläche, eine Länge (L1, L4) im Bereich von 0,5 mm bis 0,8 mm, insbesondere 0,55 mm bis 0,75 mm, insbesondere 0,65 mm oder 0,6 mm, und/oder eine Breite (B1, B2) im Bereich von 0,25 mm bis 0,35 mm, insbesondere 0,3 mm aufweist

9. Chirurgisches Instrument (1) nach einem der Schutzansprüche 1 bis 8 wobei die Außenfläche des stirnseitig geschlossenen distalen Endes (5) der Hohlnadel (2) zumindest abschnittsweise gekrümmt ist, wobei die Außenfläche optional zumindest abschnittsweise einen Krümmungsradius (R1) im Bereich von 0,15 mm bis 0,35 mm, vorzugsweise 0,2 mm bis 0,3 mm aufweist.

10. Chirurgisches Instrument (1) nach einem der Schutzansprüche 1 bis 9 wobei der Öffnungsrand der Hohlnadel (2), insbesondere in Draufsicht betrachtet, zumindest abschnittsweise gekrümmt ist mit einem Krümmungsradius (R2) im Bereich von 0,1 mm bis 0,2 mm, vorzugsweise 0,15 mm.

11. Chirurgische Vorrichtung, insbesondere Vitrektomie-Vorrichtung, umfassend
ein chirurgisches Instrument (1) nach einem der Schutzansprüche 1 bis 10, und ferner eine Laserlichtquelle, insbesondere eine Nd:YAG Laserlichtquelle, zur Einkopplung von Laserlicht, insbesondere Laserpulsen, in den Hohlraum (4, 8), insbesondere in die Kammer (8), der Hohlnadel (2), insbesondere über eine zumindest abschnittsweise im Inneren der Hohlnadel (2) angeordneten Lichtleiter (10), wobei die Laserlichtquelle, insbesondere eine Steuereinheit zur Steuerung der Laserlichtquelle, dazu eingerichtet ist, Laserlicht mit einer Pulsfrequenz im Bereich von 40 Hz bis 60 Hz zu erzeugen, und umfassend optional des Weiteren
eine mit dem inneren Hohlraum (4, 8) der Hohlnadel (2) gekoppelte oder koppelbare Absaugeinheit, wobei die Absaugeinheit vorzugsweise derart eingerichtet und betreibbar ist, dass der Hohlraum (4, 8), insbesondere die Kammer (8), der Hohlnadel (2) mit Unterdruck beaufschlagbar ist, insbesondere mit einem Unterdruck im Bereich von 0,2 bar bis 0,4 bar, vorzugsweise von 0,25 bar bis 0,35 bar, weiter vorzugsweise von 0,26 bar bis 0,33 bar.

12. Elektronische Steuereinrichtung mit einer Steuerschnittstelle zur steuerungstechnischen Kopplung mit einem chirurgischen Instrument (1) nach einem der Schutzansprüche 1 bis 10 und/oder einer chirurgischen Vorrichtung nach Anspruch 11, umfassend Steuermittel deren Ausführung bei Betrieb der Steuereinrichtung bei dem chirurgischen Instrument (1) bzw. der chirurgischen Vorrichtung die folgenden Verfahrensschritte bewirken:
Erzeugen von Laserpulsen, insbesondere mit einer Laserpulsfrequenz im Bereich von 40 Hz bis 60 Hz, wobei die Laserpulse über einen Lichtleiter (10) oder eine Lichtoptik in den inneren Hohlraum (4, 8), insbesondere die Kammer, der Hohlnadel (2) eingekoppelt werden, und an einer Lichtaustrittsseite des Lichtleiters (10) bzw. der Lichtoptik austreten, derart, dass in einer bzw. der im Inneren der Hohlnadel (2) ausgebildeten Kammer (8), die mit einer gemäß einer chirurgischen Vorrichtung (1) der Schutzansprüche 1 bis 10 ausgebildeten Vitrektomieöffnung (6) fluidtechnisch kommunizierend ausgebildet ist, befindliches Glaskörpermaterial (9) des Auges durch Einwirkung der Laserpulse zerkleinert, insbesondere zerschnitten, wird, und
Beaufschlagen des inneren Hohlraums (4, 8), insbesondere der Kammer (8), der Hohlnadel (2) mit Unterdruck, insbesondere im Bereich von 0,2 bar bis 0,4 bar, vorzugsweise von 0,25 bar bis 0,35 bar, weiter vorzugsweise von 0,26 bar bis 0,33 bar, derart, dass das durch die Laserpulse zerkleinerte Glaskörpermaterial (11) abgesaugt, und gleichzeitig weiteres Glaskörpermaterial (9) in die Kammer (8) eintritt wenn die entsprechend nach einer chirurgischen Vorrichtung (1) nach einem der Schutzansprüche 1 bis 10 ausgebildete Vitrektomieöffnung (6) im Glaskörper eines Auges positioniert ist.
